# EUROPEAN PATENT SPECIFICATION

(11) **EP 3 500 207 B1**
(45) Date of publication and mention of the grant of the patent: **17.02.2021**
(21) Application number: 17772141.2
(22) Date of filing: 21.08.2017
(51) Int. Cl.: A61C 5/50, A61C 5/55

(54) **CARRIER BASED DENTAL ROOT CANAL OBTURATOR AND MANUFACTURING METHOD THEREOF**
TRÄGERBASIERTER ZAHNWURZELKANALOBTURATOR UND VERFAHREN ZU SEINER HERSTELLUNG
OBTURATEUR DE CANAL RADICULAIRE DENTAIRE SUR SUPPORT ET MÉTHODE POUR SA FABRICATION

(30) Priority: 19.08.2016 US 201662377508 P
(43) Date of publication of application: 26.06.2019
(73) Proprietor: DENTSPLY SIRONA Inc., York, PA 17401-2991 (US)
(72) Inventor: LI, Nathan Y., Lancaster, CA 93534 (US)
(74) Representative: Schiener, Jens
(86) International application number: PCT/US2017/047857
(87) International publication number: WO 2018/035535

(56) References cited:
- US-A1- 2005 003 328
- US-A1- 2008 014 554
- US-A1- 2014 272 802

## Description

### BACKGROUND OF THE INVENTION

### Field of the Invention

The present invention is directed to materials for filling dental root canals, and in particular dental root canal filling cones or points.

### Description of Related Art

Dental root canal treatment generally involves three stages: shaping, cleaning and obturation (generally involving filling and sealing). The purpose of performing dental root canal treatment is to remove infected dental pulp tissue inside the pulp chamber and root canals, and to fill/seal the vacant space with a biocompatible material. More specifically, the ultimate objective of root canal treatment is to eliminate the infection inside the dental root system and to tightly seal or obturate, in three dimensions (3-D), the tiny openings at the end of the root canal, (referred in the profession as an apex). Failure to completely seal the apex or the root canal in 3-D leads to micro-leakage, which will lead to future bacteria colonization inside the root canal system, and re-infection and possible loss of the tooth. Micro-leakage is the most common cause of tooth failure.

Heretofore, root canal treatment processes involve placement of a root canal filling and/or sealing point or cone in a prepared root canal to plug the root canal, ideally in a manner to eliminate micro-leakage. Hereinafter, the term "point" and "cone" will be used interchangeably to refer to dental root canal filler/sealer. In the past twenty-plus years, leading dentists and scientists have improved and revolutionized the shaping and cleaning part of the root canal treatment process. But the basic filling technique still lags behind due to antiquated manufacturing process dated more than 50 years ago. The existing filling points and the process of application thereof do not lend themselves well to providing a good seal of the root canal apex.

The most commonly used root canal filling material for many years is a biocompatible latex compound commonly called Gutta Percha (GP), which comprises polyisoprene, or trans-polyisoprene with a chemical composition of 1,4-trans-polyisoprene (TPI). Other thermoplastic polyurethane based compounds may be adopted. The filling material is made into cone/point shape with different diameter sizes at the tip of the cone. When heated, the material will soften to flow at rather low temperature settings, typically starting at 60 to 80 degrees Celsius. In this soften state, users (dentists or specialists known as endodontists) can mold/pack filling material all the way into root canal system for optimum obturation, filling and sealing effects. The filling material is chemically inert, and is therefore more biocompatible. Gutta Percha also hold its dimension quite well when change from heated liquid alpha phase to cooled solid beta stage.

The way to use Gutta Percha to fill/seal the root canal is to make it into a tapered cone shape "cone" or "point", commonly called Gutta Percha point or cone. Heretofore, root canal filling points are formed of a filling material that is shaped into slender cones each having a small taper angle (e.g., 5-10 degrees). Each point is made into a particular tapered or conical shape that matches the shaping instrument (file) used by dentists to shape a root canal cavity for subsequent filling. The traditional way of making these points is by manual labor, specifically hand rolling Gutta Percha material into points to match shaping files. The Gutta Percha material needs to be softened first with higher temperature. Then being rolled into the point while being cooled to hold the final shape. This method of making the points has been in existence for over 50 years without much change. It is grossly inaccurate and risks material contamination since it is mostly handled by human hands.

The last step of dental root canal treatment is to use a biocompatible material such as Gutta Percha filling and/or sealing shaped and disinfected root canal space. One filling device currently used is called carrier based root canal obturator. Generally, it is a small quite rigid cone shaped stick (called carrier core) with a small handle to be held with thumb and index finger. The front portion of the stick is coated with Gutta Percha material. It is preheated with a small oven to soften up the outer Gutta Percha layer, and then held with thumb and finger to insert it all the way to the very end of the root canal space.

US Patent No. 5,089,183 discloses a method of manufacturing appliances for use in filling endodontically prepared root canals with filler material, which involves inserting a shaft of a carrier into an uncured Gutta Percha material provided in a cavity of a block, heating and allowing the material to adhere to the carrier shaft. This process is low throughput, as it adds further complication to the making of a filler point for root canal.

There are several short comings with this line of devices. The biggest shortcoming is the size inconsistency of this type of obturator, in particular, carrier core size. In order for root canal space to be filled and sealed perfectly, the obturator must have a perfect dimension matching with the root canal file used to shape the root canal. The current carrier based obturator has size either too big that it can't reach into the end of fully shaped root canal space or too small that it got pushed beyond the end of fully shaped root canal space. This will result in poorly sealed root canal space and fail to prevent bacteria reentry into human body system. Other than the size inconsistency of this type of obturator as a big problem, production process low efficiency is another major problem. The third major problem is the separation of surface coated material from the core itself when being inserted into root canal space during treatment process (the carrier core could easily break through the softer surface coating layer of Gutta Percha. It is often a challenge in inserting a CBO into a root canal that is curved. And the current core carrier is very brittle, so it could easily break during treatment process.

US Patent No. 7,942,673 attempted to provide a stopping mechanism on a dental obturator, to limit the extent a carrier core can be inserted in a root canal. The obturators are disclosed as being able to bind at a particular distance into the canal, thereby precluding possible damage at the end of the canal. However, currently, there does not appear to be any commercial dental obturator product in the market based on this concept. As disclosed, multiple "stoppers" are circumferentially located close to a thin end of the carrier core, at an axial location corresponding to the small space available at or near the bottom (apex) of the root canal. It does not appear that this patent disclosure provided an effective feature, as the limited space in the root canal would not be able to effectively receive a carrier core having such stoppers. US 2005/0003328 A1 discloses an endodontic post and an obturating system.

There remains an unresolved need to provide an improved carrier based dental obturator (root canal filling points/cones) that overcomes the above-noted drawbacks in the prior art.

### SUMMARY OF THE INVENTION

The present invention relates to the subject matter of claim 1 to 14, and in particular provides an improved carrier based dental obturator (root canal filling points/cones) according to claims 1 or 4 that overcomes the above-noted drawbacks in the prior art. The present invention provides an improved structure of the carrier, which also improves production processes, resulting in the skin or surface coating layer of sealing/filling material being more efficiently and more precisely applied to and supported by the carrier. The present invention also provides a method of manufacturing a carrier based dental obturator.

The present invention will be described herein-below in reference to the example of carrier based obturator (or root canal filling points/cones) made of endodontic filling material including what is known as Gutta Percha, for example. However, it is understood that the present invention could be applied to root canal filling points based on other types of endodontic filler materials, natural or synthetic, currently known or future discovered, without departing from the scope of the present invention.

### BRIEF DESCRIPTION OF THE DRAWINGS

For a fuller understanding of the nature and advantages of the invention, as well as the preferred mode of use, reference should be made to the following detailed description read in conjunction with the accompanying drawings. In the following drawings, like reference numerals designate like or similar parts throughout the drawings.
Fig. 1 is schematic sectional view of a human tooth, schematically showing two root canals into which a carrier based obturator of the present invention can be used for root canal treatment.
Fig. 2 is a perspective view of a carrier based obturator in accordance with one embodiment of the present invention.
Fig. 3A-3I are various views illustrating a carrier based obturator in accordance with one embodiment of the present invention.
Fig. 4A-4G are various views illustrating a carrier based obturator in accordance with another embodiment of the present invention.

### DETAILED DESCRIPTION OF THE PREFERRED EMBODIMENTS

This invention is described below in reference to various embodiments with reference to the figures. While this invention is described in terms of the best mode for achieving this invention's objectives, it will be appreciated by those skilled in the art that variations may be accomplished in view of these teachings without deviating from the spirit or scope of the invention.

The present invention provides an improved carrier based dental obturator (root canal filling points/cones) that overcomes the above-noted drawbacks in the prior art. The present invention provides an improved structure of the carrier, which also improves production processes, resulting in the skin or surface coating layer of sealing/filling material being more efficiently and more precisely applied to and supported by the carrier.

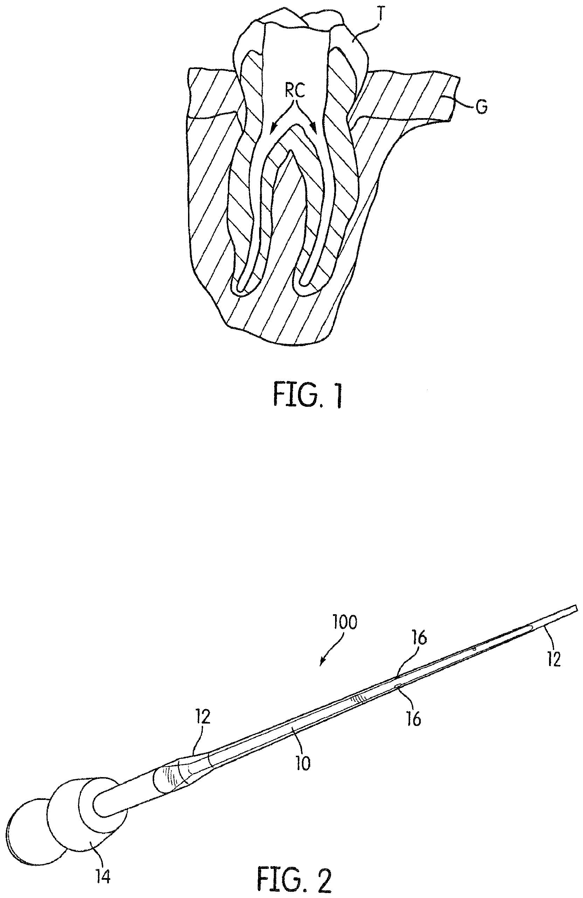

Fig. 1 is a schematic sectional view of a human tooth T and gum G, showing two exemplary root canals RC into which a carrier based obturator of the present invention can be used for root canal treatment.

The present invention will be described herein-below in reference to the example of carrier based obturator (or root canal filling points/cones) made of endodontic filling material including what is known as Gutta Percha, for example. However, it is understood that the present invention could be applied to root canal filling points based on other types of endodontic filler materials, natural or synthetic, currently known or future discovered, without departing from the scope and spirit of the present invention.

In one aspect, the invention provides more precise carrier based obturator with stopping pegs built into the core, and to apply the outer skin layer in a more precise fashion (e.g., using injection molding or dipping processes). A stopping mechanism is built in the carrier core of the carrier based obturator to prevent accidental over fill - pushing the obturation beyond root canal space. In another aspect of this invention, an injection molding process is developed to make the carrier core and/or the carrier based obturator (i.e., injection molding obturation material over a carrier core regardless of how the latter is made). Alternatively, the present invention provides a new approach to form the surface obturation layer by dipping. This improves throughput to produce quality products with low rejection rate. In a further aspect, the core body is made less brittle so that there will be less breakage during the insertion into root canal space, and to make coated surface layer better adhere to the core to prevent separation / stripping.

As an initial note, the overall size of the carrier based obturator, i.e., the carrier core plus surface coating layer (e.g., Gutta Percha surface coating layer) shall be about the same size (or slightly smaller size, or slightly larger size) as the root canal file being used to shape the root canal. Therefore, the carrier core shall be much smaller than the root canal file so it has room to add Gutta Percha surface coating layer.

The present invention provides a distribution of protrusions, including bumps, pegs or stoppers, such as several pairs of half round (or semi-spherical) pegs, along the surface of this carrier core. For example, one or more pegs, disposed at an axial location towards the front, small end part of a tapered carrier core body. At a second axial location a few millimeters away from the first pair is another one or more pegs and has a 90 to 180-degree offset angle from the first pair. When measuring the carrier core diameter where the pair of pegs are located (peg height + core diameter + peg height), it shall be equal to or slightly smaller than the file diameter used for shaping at same location. By having this design, the doctor operator will not be able to push the carrier core beyond the end of root canal space shaped by the corresponding file because the pair of pegs on the carrier core surface will get stuck inside root canal space. This design also ensure that the carrier core is located in the center of root canal space to provide even distribution of the skin layer which is the true, effective or active obturation material. These precision-made pegs function as "stoppers", which can be in any geometrical shapes (e.g., pyramidal shaped, pin shaped, etc.), at multiple locations. Each axial location can have pair of "stoppers" at 180-degree from each other, or three or more, such as three at 120-degree from each other. This carrier core shall be made as semi-rigid to rigid so the carrier based obturator can have some degree of flexibility with adequate rigidity to allow bending for easy and controlled insertion into a bending root canal space (i.e., to navigate into a prepared root canal space). The carrier core may be made by a number of processes, including casting, stamping, molding, injection molding, 3D printing, etc. In accordance with one embodiment of the present invention, the carrier core is precisely made by injection molding.

Fig. 2 is a perspective view of a carrier based obturator 100 in accordance with one embodiment of the present invention. In accordance with the present invention, the carrier core 10 is provided with predefined surface features including axially and/or circumferentially distributed protrusions 16 (e.g., bumps, pegs or stoppers) along the surface of the carrier core 10, to provide not only a stopping function, but also a support for the coating layer 12 to guide insertion and seating of the carrier based obturator 100 in the root canal RC. The coating layer 12 extends beyond the distal end/tip of the carrier core 10. In one embodiment, the carrier core 10 includes a body (e.g., a conical body having a generally flat surface in the axial direction, having a predefined conical taper) and surface protrusion features 16 defined at the surface of the core body which include one or more discrete protrusions from the surface of the core body at two or more axial locations.

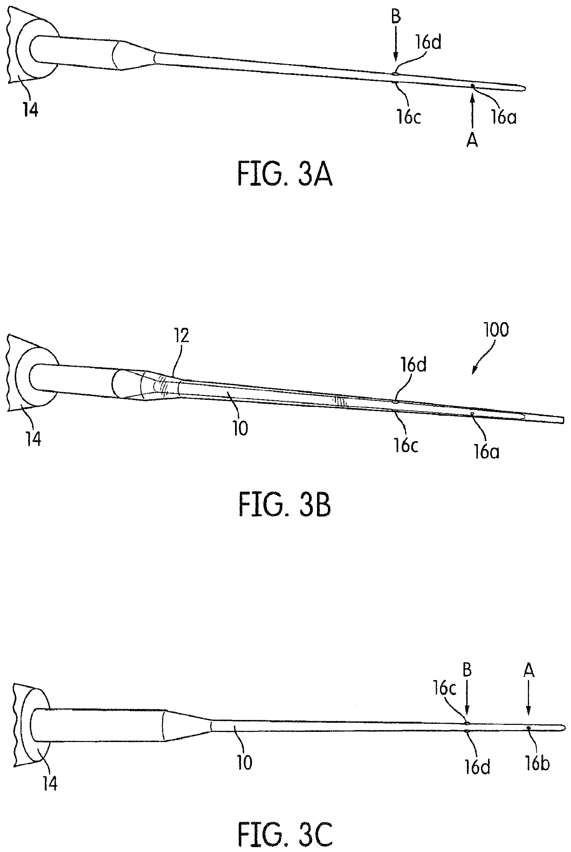

Figs. 3A to 3I are further views illustrating a carrier based obturator 100 in accordance with one embodiment of the present invention. The obturator 100 comprises a generally overall conical shape structure, comprising a handle 14, a carrier core 10, and a surface coating layer 12 of root canal filling and/or sealing material, which is a heat flowable material, such as Gutta Percha. The carrier core 10 has a thick or large tail end and a tapered thin or small tip end, which has a taper angle that fits in the apex end of a prepared root canal cavity (the taper angle at the apex of the cavity being defined using a tapered file tool known in the dentistry field). The diameter of each diametric section along the longitudinal axis of the body is substantially circular, up to the large tail end. Extending beyond the large tail end of the carrier core 10 is the handle 14, which may have the structure as shown in the drawings, or a flat tab. An identification indicium (e.g., alphanumeric) may be provided on the handle 14 (or on the flat surface of the tab), to facilitate the user (dentist) to distinguish the particular configuration of the obturator (e.g., the indicia corresponds to a particular size, taper angle, filling/coasting material, etc.).

The general dimensions of the carrier core 10 and the Gutta Percha material layer 12 of the obturator 100 may be referenced from the drawings.

During a dental root canal treatment process, the obturator body (carrier core 10 and filling/sealing material layer 12) is heated and inserted into the prepared root canal cavity RC. The handle/tab 14 (along with excessive section of the core 10 that is not needed) can be removed by cutting before or after insertion. Additional heat may be applied to the large end of the filling material 12 using a heating tool (e.g., a heat gun). As the Gutta Percha material softens under the applied heat, the material flows in the root canal cavity to fill the root canal RC. Ideally, sufficient heat reaches the small end of the filling cone to flow the material 12 to completely fill the apex of the root canal cavity RC.

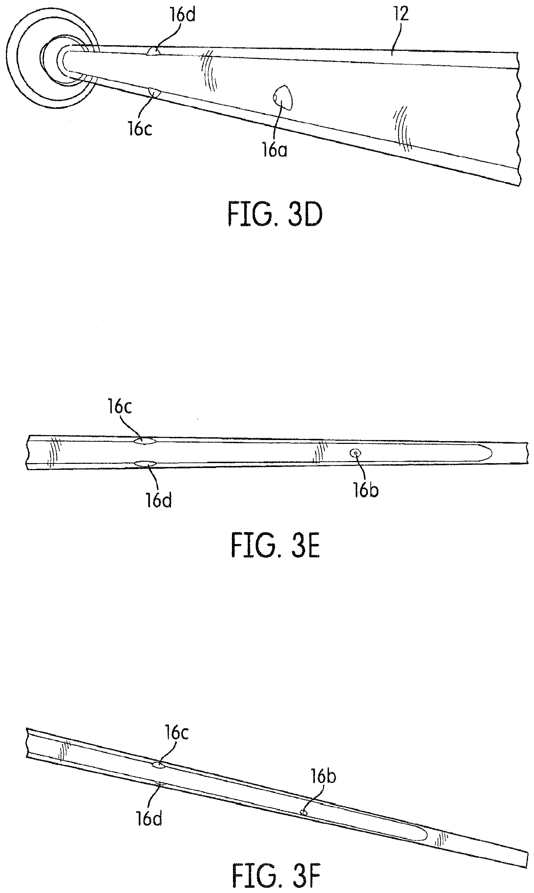

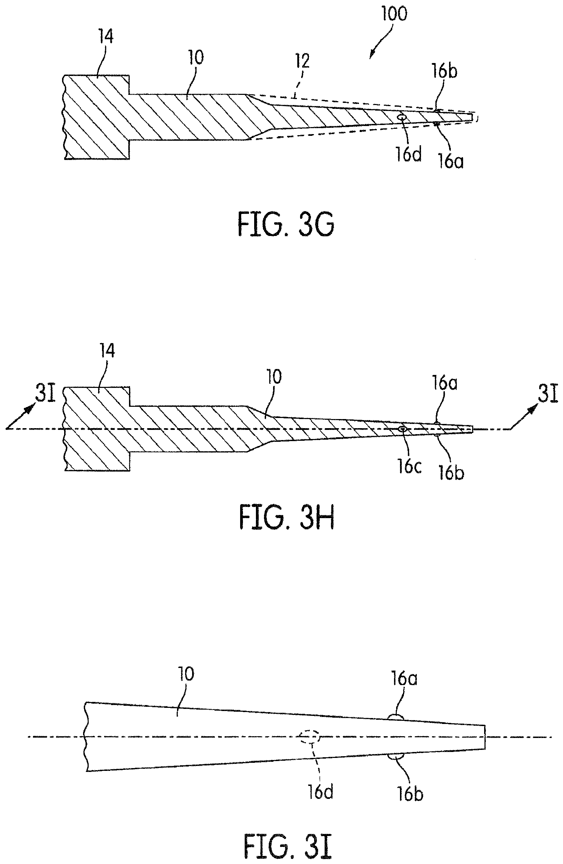

In the embodiment illustrated in Figs. 3A to 3I, in reference to the small end of the carrier core 10 (first "0" axial location being closest to the small end of the carrier core 10, and second, third and fourth axial locations in that order), a first pair of protrusions 16a/16b are provided at a first axial location A, and a second pair of protrusions 16c/16d are provided at a second location B of the carrier core body, offset by 90° circumferentially in relation to the first pair of protrusions 16a/16c. Fig. 3A shows the view of the carrier core 10 with the protrusion 16a in view, but with the protrusion 16b hidden from view. Figs. 3B, 3D are similar views of the carrier core 10, with the filling material layer 12 shown. Figs. 3C, 3E and 3F are views of the carrier core 10 rotated 180 degrees about its axis from the view in Fig. 3A. Fig. 3I is a sectional view of the carrier core 10 taken alone line 31-31 in Fig. 3H. Figs. 3A and 3B provide the diameters of the carrier core 10 and the diameters of core+GP material at various axial locations of the carrier core 10. Fig. 3C provides the distances of axial locations from the axial location "0", in mm. The coating layer 12 extends beyond the distal end/tip of the carrier core 10.

While the illustrated embodiments show two pairs of protrusions at two axial locations, it is well within the scope of the present invention to have more pairs of protrusions at more than two axial locations.

In another embodiment of a carrier based obturator 100', the surface features include a single discrete protrusion 16' at each axial location along the carrier core body 10, with relative circumferential position offset by a predetermined angle between axially adjacent protrusions. The individual protrusions 16' define discrete points of contact when the carrier core body 10 is inserted into the root canal RC. The individual protrusions 16' above the surface of the carrier core body are discrete, as they do not interconnect to form a protrusion resembling a band or line of contact with the root canal walls.

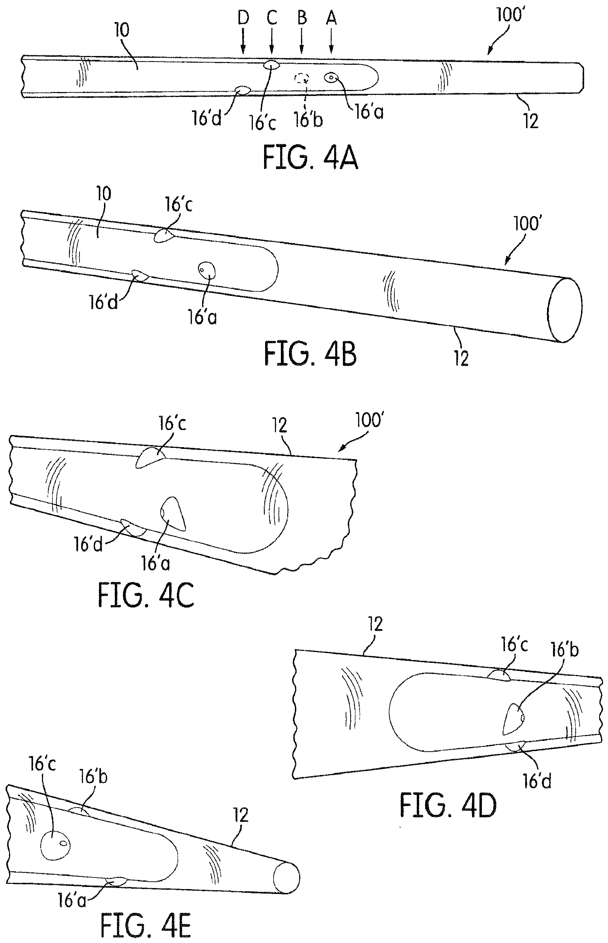

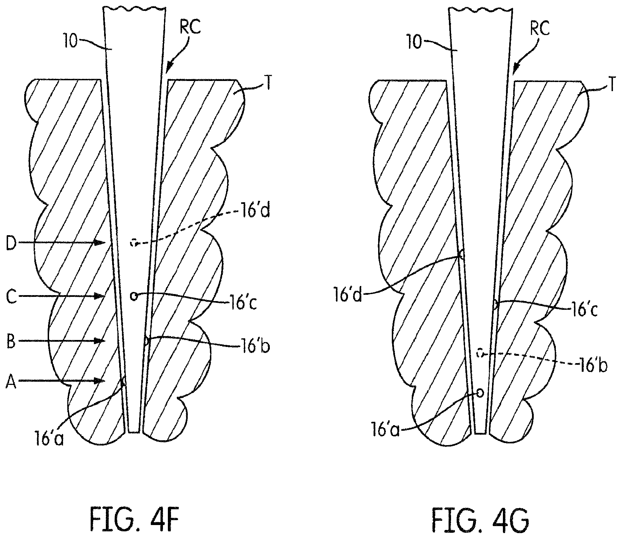

Referring to the embodiment of the carrier based obturator in Figs. 4A to 4G, a first protrusion 16'a at a first axial location A and a second protrusion 16'b at a second axial location B are offset by 180° circumferentially; a third protrusion 16'c at a third axial location C and the second protrusion 16'b are offset by 90° circumferentially, and the third protrusion 16'c and a fourth protrusion 16'd at a fourth axial location D are offset by 180° circumferentially. Figs. 4A to 4C show the view of the carrier core 10 with the protrusion 16'a in view, but with the protrusion 16'b hidden from view. Figs. 4D and 4E are views of the carrier core 10 rotated 90 degrees about its axis from the view in Fig. 4A. Figs. 4F and 4G are schematic sectional views of the carrier core 10 of Figs. 4A to 4E inserted into a root canal RC, with the carrier core 10 in Fig. 4G rotated by 90 degrees from Fig. 4F. The coating layer 12 extends beyond the distal end/tip of the carrier core 10.

In one embodiment, the heights of the protrusions are substantially flush with the external surface (exposed side) of the coating material 12 (e.g., Gutta Percha).

In one embodiment, the heights of the protrusions 16 are the same on the tapered body of the carrier core. In another embodiment, the heights may be different.

In one embodiment, the protrusions may be defined by a semi-sphere, semi-ovoid, tetrahedron, and/or, pyramidal shaped protrusions. Other geometrical shapes may be defined with a generally convex surface for contact with the root canal walls.

In one embodiment, the surface coating of the carrier based obturator is made of Gutta Percha, or other appropriate dental filling and/or sealing material, natural or synthetic.

While the illustrated embodiments show four protrusions at four axial locations, it is well within the scope of the present invention to have more protrusions at more axial locations.

It is also well within the scope of the present invention to combine the embodiments in Figs. 3 and 4, that is, with pairs of protrusions at various axial locations, as well as additional single protrusions at various axial locations.

Further, the surface coating layer of the obturator may include nanoparticles in a matrix material of the surface coating layer, e.g., Gutta Percha, of the carrier based obturator to improve thermal conductive characteristics, as disclosed in U.S. Patent No. 9,192,545, which is issued to the inventor of the present : invention. The characteristic size (or the statistical average size) of the nanoparticles is on the order of 1000 nm or less (e.g., the particle diameter if spherical). In one embodiment, the characteristic size is 500 to 1000 nm. Some of the particles may have characteristic size on the order of 100 nm or less. According to nano-technology, nanoparticles exhibit properties that are not found in bulk samples of the same material. At the nano-scale, the physics of nanoparticles is such that their properties are different from the properties of the same material in larger, bulk form. The characteristic size of the particles does not have to be uniform. All particles may be generally or substantially the same size, or have random sizes within the prescribed size range. [0001] The particles may be made of high heat conductive metal, non-metal, organic or inorganic materials, including without limitations Zinc Oxide, Magnesium Silicate, gold, silver, titanium, diamond, etc. The matrix of base filling material may include natural or synthetic heat flowable polymeric materials that are bio-inert or bio-compatible when disposed in dental root canals, which may include rubber, thermoplastic or other polymeric materials. Rubber material may include trans-polyisoprene based material, such as Gutta Percha. In a specific embodiment, metallic nanoparticles are dispersed in dental Gutta Percha material. For example, Zinc Oxide nanoparticles are added to a matrix of dental Gutta Percha material to form a composite root canal filling material. Zinc Oxide nanoparticles in powder form are commercially available, with particle sizes in 700-900nm, or smaller sizes of 100 nm, 75 nm, 50 nm, and 20 nm. Other sizes of nanoparticles could also work in accordance with the present invention. The inventive carrier based obturator can be made with the filling material having metallic and non-metallic nanoparticles described above, using traditional roll processes or using the inventive injection molding process disclosed in U.S. Patent Publication No. 2014/0315155A1, which was filed by the inventor of the present invention.

Concerning the taper of the obturator, it may have a single taper angle for substantially its entire length (i.e., an overall single-taper obturator as shown), or a different taper angle for different longitudinal sections along its length (i.e., multi-taper cones, e.g., disclosed in U.S. Patent Application Publication No. US2014/0272802A1, which is a U.S. patent application filed by the inventor of the present : invention). In one embodiment, the multi-taper Gutta Percha cones each has a generally axisymmetric conical structure, wherein at least a section along the length of the Gutta Percha cone has a tapered structure, wherein the taper angle varies progressively in the axial direction to result in a multi-taper or variable taper conical structure. In one embodiment, the taper angles vary in small, discrete incremental steps along the length of the point, thus forming a structure having adjoining conical sections having different and discretely varying tapers at different axial sections along the length.

In a further embodiment, the carrier core body may have a single tapered conical shape, and the heights and axial locations of the protrusions can be selected to define a carrier based obturator having an overall multi-tapered conical shape. Given the protrusions are flush with exterior surface of the surface coating layer, the surface coating layer would have a multi-tapered conical profile. The highest points of the protrusions (i.e., the contact points with the root canal wall) essential can be used to define the locus of the overall shape of the obturator.

In another aspect of the present invention, the protrusions 16 of the carrier core 10 provide a means to set the thickness for over-molding the surface coating layer 12 (e.g., Gutta Percha material) onto the carrier core. The carrier core 10 can be received in the cavity of the mold conforming to the desired shape and dimension of the desired carrier based obturator, with the protrusions touching the cavity walls (as if touching a root canal cavity), thereby defining the thickness and geometry of the surface coating layer to be molded onto the carrier core, to result in quality carrier based obturator having precise dimensions and geometry conforming to obturation files used by orthodontists to open a root canal for obturation treatment.

This inventor has filed for patent application (U.S. Application No. 14/181621; published as US 20140315155 A1) directed to a technology for precision molded root canal obturation cones. Products made from this technology has been on the market and has gained international recognition as the most precise root canal obturation cones available today. The inventor improves on this technology to make the precisely molded carrier based obturator with stopping mechanism built in the carrier core to prevent accidental over fill - pushing the obturation beyond root canal space. The inventor also improves on this technology to make new carrier based obturator, with Gutta Percha and any other known or future root canal obturation material, in more precise fashion.

Referring to US 20140315155 A1, the carrier core may be precision molding using a first mold to form a carrier core body having the protrusions discussed above. After the precision molded carrier core is made (e.g., from its own mold), it is placed into a second mold for injecting molding the carrier surface skin layer onto the carrier core with precision. The mold cavity in the second mold for the surface skin layer is precision made to match the size of the root canal shaping file. The two pairs of pegs serve as a positioning device within the mold cavity for second round mold injection process to ensure the carrier core is centrally located with the mold cavity and the skin layer is evenly distributed around the core. Another way to explain it, the carrier core's half round pegs will hold the core inside the mold cavity like an inlay piece (the molding process may be referred to as inlay molding or overmolding). Then finally the melted Gutta Percha or other desirable material is molded onto the core surface. The pegs act like spacers inside the mold cavity, allowing the skin layer of Gutta Percha to be molded over the carrier core.

In another embodiment of this invention, the surface skin layer may be coated over the carrier core that is provided with pegs or stoppers as disclosed above, by dipping the carrier core into a bath of molten obturation material. Gutta Percha or other biocompatible root canal material is heated and melted in a receptacle, e.g., a small tub, to a bath of ultra-low viscosity coating material for the surface layer (like chocolate syrup). Preferably, the viscosity of the heated material is below SAE (Society of Automotive Engineers) index/grade 35. Heating may be by inductive heating, resistive heating, Microwave, or infrared. After the chosen surface layer coating material is heated and softened to an optimum consistency of low viscosity (e.g., SAE viscosity grade 10 and 20) for an even and smooth coating of surface, a rack of pre-made (e.g., precision injection molded, stamped, cast, 3D printed, etc.) carrier cores is dipped into the heated material in the tub and then withdrawn from the tub, so that the carrier core is coated with this material. After dipping, the coating form a surface layer of generally uniform thickness. The thickness of the outer coated layer depends on various parameters, such as dipping speed, temperature, viscosity and cooling speed, size of carrier core, material of carrier core, etc. After this, the coating layer is cooled by, for example, circulating liquid Nitrogen gas to result in a stable solid pliant coating of skin layer on the carrier core. In operation, after this carrier based root canal obturator is inserted into a root canal space, the pliant coating fills the space between the carrier core and the root canal walls. This invention will substantially improve production efficiency and product accuracy comparing to the process described in US Patent No. 5089183 by Dr. William Johnson.

The carrier core surface skin layer may be made by other coating means, such as casting, thermofill molding, 3D printing, etc. with a desired root canal obturation material (e.g., Gutta Percha or other polymer), further over a carrier core having the above described stopping mechanism (protrusions, pegs or stoppers).

While the present invention has been described above in connection with the illustrated embodiments, the scope of patent invention covers all possible present and future variations and improvements that is apparent from the disclosure above. While the invention has been particularly shown and described with reference to the preferred embodiments, it will be understood by those skilled in the art that various changes in form and detail may be made without departing from the scope of the invention. Accordingly, the disclosed invention is to be considered merely as illustrative and limited in scope only as specified in the appended claims.

## Claims

1. A carrier based dental obturator (100'), comprising:
a carrier core (10) having a generally conical body;
a plurality of discrete protrusions (16'a, 16'b, 16'd, 16'd) provided at two or more axial locations on the surface of the conical body; and a layer (12) of endodontic filling material coating the carrier core, wherein the thickness of the coating layer is about the same as the height of the protrusions above the surface of the conical body, wherein the protrusions comprises a single discrete protrusion at each axial location along the carrier core body and the relative circumferential positions of the protrusions are offset by a predetermined angle between axially adjacent protrusions.

2. The carrier based dental obturator as in claim 1, wherein in reference to the distal end of the carrier core body, a first protrusion at a first axial location and a second protrusion at a second axial location are offset by 180° circumferentially.

3. The carrier based dental obturator as in claim 2, wherein a third protrusion at a third axial location and the second protrusion are offset by 90° circumferentially, and the third protrusion and a fourth protrusion at a fourth axial location are offset by 180°. circumferentially.

4. A carrier based dental obturator (100), comprising:
a carrier core (10) having a generally conical body;
a plurality of discrete protrusions (16a, 16b, 16c, 16d) provided at two or more axial locations on the surface of the conical body; and a layer (12) of endodontic filling material coating the carrier core, wherein the thickness of the coating layer is about the same as the height of the protrusions above the surface of the conical body, wherein a first pair of discrete protrusions are provided at a first axial location, and a second pair of discrete protrusions are provided at a second location of the carrier core body, offset by 90° circumferentially in relation to the first pair of protrusions.

5. The carrier based dental obturator as in any of above claims, wherein the endodontic filling material comprises Gutta Percha.

6. The carrier based dental obturator as in any of above claims, wherein the thickness of the coating is slightly below the height of the protrusions, wherein the thickness of the coating is slightly above the height of the protrusions, or the heights of the protrusions are substantially flush with the external surface of the coating material.

7. The carrier based dental obturator as in any of above claims, the heights of the protrusions are the same on the conical body of the carrier core.

8. The carrier based dental obturation as in of above claims, wherein the individual protrusions define discrete points of contact with the root canal cavity wall when the carrier core body is inserted into a root canal.

9. The carrier based dental obturation as in any of above claims, wherein the individual protrusions above the surface of the carrier core body are discrete, as they do not interconnect to form a protrusion resembling a band or line of contact with the root canal walls.

10. The carrier based dental obturator as in any of above claims, wherein the protrusions are defined as a semi-spherical, semi-ovoid, tetrahedron, and/or, pyramidal shaped protrusions.

11. The carrier based dental obturator as in any of above claims, wherein the carrier core body has a single taper along the section to be inserted into a root canal.

12. The carrier based dental obturator as in any of claims 1 to 10, wherein the carrier core body has multiple tapers along the axial direction of the carrier core body.

13. The carrier based dental obturator as in any of above claims, wherein the carrier core body is made of a material that may include at least a polymer, such as polyethylene, polyurethane, polyvinyl chloride.

14. A method of manufacturing a carrier based dental obturator (100') comprising:
providing a mold having at least a mold cavity conforming to the carrier based obturator;
providing a carrier core (10) having a generally conical body, wherein a plurality of discrete protrusions; (16'a, 16'b, 16'c, 16'd) are provided at two or more axial locations on the surface of the conical body; and
molding a layer (12) of endodontic filling material coating the carrier core, wherein the thickness of the coating layer s about the same as the height of the protrusions above the surface of the conical body,
wherein the protrusions comprises a single discrete protrusion at each axial location along the carrier core body, and wherein the relative circumferential positions of the protrusions are offset by a predetermined angle between axially adjacent protrusions.

## Patentansprüche

1. Trägerbasierter Dentalobturator (100'), umfassend:
einen Trägerkern (10) mit einem im Allgemeinen konischen Körper; eine Vielzahl diskreter Vorsprünge (16'a, 16'b, 16'd, 16'd), die an zwei oder mehr axialen Stellen auf der Oberfläche des konischen Körpers vorgesehen sind; und eine Schicht (12) aus endodontischem Füllmaterial, die den Trägerkern bedeckt, wobei die Dicke der Deckschicht ungefähr der Höhe entspricht, um welche die Vorsprünge über die Oberfläche des konischen Körpers hinausragen, wobei die Vorsprünge einen einzelnen diskreten Vorsprung an jeder axialen Stelle entlang des Trägerkörpers umfassen und die relativen Umfangspositionen der Vorsprünge durch einen vorbestimmten Winkel zwischen axial nebeneinanderliegenden Vorsprüngen versetzt sind.

2. Trägerbasierter Dentalobturator nach Anspruch 1,
wobei in Bezug auf das distale Ende des Trägerkernkörpers ein erster Vorsprung an einer ersten axialen Stelle und ein zweiter Vorsprung an einer zweiten axialen Stelle umfänglich um 180° versetzt sind.

3. Trägerbasierter Dentalobturator nach Anspruch 2,
wobei ein dritter Vorsprung an einer dritten axialen Stelle und der zweite Vorsprung umfänglich um 90° versetzt sind und der dritte Vorsprung und ein vierter Vorsprung an einer vierten axialen Stelle umfänglich um 180° versetzt sind.

4. Trägerbasierter Dentalobturator (100), umfassend:
einen Trägerkern (10) mit einem im Allgemeinen konischen Körper;
eine Vielzahl diskreter Vorsprünge (16a, 16b, 16c, 16d), die an zwei oder mehr axialen Stellen auf der Oberfläche des konischen Körpers vorgesehen sind; und eine Schicht (12) aus endodontischem Füllmaterial, die den Trägerkern bedeckt, wobei die Dicke der Deckschicht ungefähr der Höhe entspricht, um welche die Vorsprünge über die Oberfläche des konischen Körpers hinausragen,
wobei ein erstes Paar diskreter Vorsprünge an einer ersten axialen Stelle vorgesehen ist und ein zweites Paar diskreter Vorsprünge an einer zweiten Stelle des Trägerkernkörpers vorgesehen ist, wobei das zweite Paar von Vorsprüngen in Bezug auf das erste Paar von Vorsprüngen umfänglich um 90° versetzt ist.

5. Trägerbasierter Dentalobturator nach einem der vorhergehenden Ansprüche, wobei das endodontische Füllmaterial Guttapercha umfasst.

6. Trägerbasierter Dentalobturator nach einem der vorhergehenden Ansprüche, wobei die Dicke der Beschichtung geringfügig kleiner ist als die Höhe der Vorsprünge, wobei die Dicke der Beschichtung geringfügig größer ist als die Höhe der Vorsprünge oder die Höhe der Vorsprünge im Wesentlichen bündig mit der externen Oberfläche des Beschichtungsmaterials ist.

7. Trägerbasierter Dentalobturator nach einem der vorhergehenden Ansprüche, wobei die Höhen der Vorsprünge auf dem konischen Körper des Trägerkerns gleich sind.

8. Trägerbasierter Dentalobturator nach einem der vorhergehenden Ansprüche, wobei die einzelnen Vorsprünge diskrete Kontaktpunkte mit der Wurzelkanalhohlraumwand definieren, wenn der Trägerkernkörper in einen Wurzelkanal eingeführt wird.

9. Trägerbasierter Dentalobturator nach einem der vorhergehenden Ansprüche, wobei die einzelnen über die Oberfläche des Trägers hinausragenden Vorsprünge diskret sind, da sie sich nicht miteinander verbinden, um einen einer Berührungslinie mit den Wurzelkanalwänden ähnelnden Vorsprung zu bilden.

10. Trägerbasierter Dentalobturator nach einem der vorhergehenden Ansprüche, wobei die Vorsprünge als halbkugelförmige, halb-ovoide, tetraederförmige und/oder pyramidenförmige Vorsprünge definiert sind.

11. Trägerbasierter Dentalobturator nach einem der vorhergehenden Ansprüche, wobei der Trägerkernkörper eine einzige Verjüngung entlang des in einen Wurzelkanal einzuführenden Abschnitts aufweist.

12. Trägerbasierter Dentalobturator nach einem der Ansprüche 1 bis 10, wobei der Trägerkernkörper mehrere Verjüngungen entlang der axialen Richtung des Trägerkernkörpers aufweist.

13. Trägerbasierter Dentalobturator nach einem der vorhergehenden Ansprüche, wobei der Trägerkernkörper aus einem Material hergestellt ist, das zumindest ein Polymer, wie beispielsweise Polyethylen, Polyurethan, Polyvinylchlorid, beinhalten kann.

14. Verfahren zur Herstellung eines trägerbasierten Dentalobturators (100'), umfassend:
Bereitstellen einer Form mit zumindest einem Formhohlraum,
die dem trägerbasierten
Obturator entspricht;
Bereitstellen eines Trägerkerns 1 (10) mit einem im Allgemeinen konischen Körper, wobei eine Vielzahl diskreter Vorsprünge (16'a, 16'b, 16'c, 16'd) an zwei oder mehr axialen Stellen auf der Oberfläche des konischen Körpers vorgesehen sind; und Formen einer Schicht (12) aus endodontischem Füllmaterial, die den Trägerkern bedeckt, wobei die Dicke der Deckschicht ungefähr der Höhe entspricht, um welche die Vorsprünge über die Oberfläche des konischen Körpers hinausragen,
wobei die Vorsprünge einen einzelnen diskreten Vorsprung an jeder axialen Stelle entlang des Trägerkernkörpers umfassen und wobei die relativen Umfangspositionen der Vorsprünge durch einen vorbestimmten Winkel zwischen axial nebeneinanderliegenden Vorsprüngen versetzt sind.

## Revendications

1. Obturateur dentaire avec support (100'), comprenant :
un noyau de support (10) ayant un corps généralement conique ;
une pluralité de protubérances discrètes (16'a, 16'b, 16'd, 16'd) prévues au niveau d'au moins deux emplacements axiaux sur la surface du corps conique ;
et une couche (12) de matériau de remplissage endodontique enrobant le noyau de support, dans lequel l'épaisseur de la couche d'enrobage est environ identique à la hauteur des protubérances au-dessus de la surface du corps conique,
dans lequel les protubérances comprennent une protubérance discrète individuelle au niveau de chaque emplacement axial le long du corps de noyau de support et les positions circonférentielles relatives des protubérances sont décalées d'un angle prédéterminé entre des protubérances axialement adjacentes.

2. Obturateur dentaire avec support selon la revendication 1, dans lequel en référence à l'extrémité distale du corps de noyau de support, une première protubérance au niveau d'un premier emplacement axial et une deuxième protubérance au niveau d'un deuxième emplacement axial sont décalées de 180° circonférentiellement.

3. Obturateur dentaire avec support selon la revendication 2, dans lequel une troisième protubérance au niveau d'un troisième emplacement axial et la deuxième protubérance sont décalées de 90° circonférentiellement, et la troisième protubérance et une quatrième protubérance au niveau d'un quatrième emplacement axial sont décalées de 180° circonférentiellement.

4. Obturateur dentaire avec support (100), comprenant :
un noyau de support (10) ayant un corps généralement conique ;
une pluralité de protubérances discrètes (16a, 16b, 16c, 16d) prévues au niveau d'au moins deux emplacements axiaux sur la surface du corps conique ;
et une couche (12) de matériau de remplissage endodontique enrobant le noyau de support, dans lequel l'épaisseur de la couche d'enrobage est environ identique à la hauteur des protubérances au-dessus de la surface du corps conique,
dans lequel une première paire de protubérances discrètes est prévue au niveau d'un premier emplacement axial, et une deuxième paire de protubérances discrètes est prévue au niveau d'un deuxième emplacement du corps de noyau de support, décalée de 90° circonférentiellement par rapport à la première paire de protubérances.

5. Obturateur dentaire avec support selon l'une quelconque des revendications précédentes, dans lequel le matériau de remplissage endodontique comprend de la gutta-percha.

6. Obturateur dentaire avec support selon l'une quelconque des revendications précédentes, dans lequel l'épaisseur de l'enrobage est légèrement inférieure à la hauteur des protubérances, dans laquelle l'épaisseur de l'enrobage est légèrement supérieure à la hauteur des protubérances, ou les hauteurs des protubérances sont sensiblement de niveau avec la surface externe du matériau d'enrobage.

7. Obturateur dentaire avec support selon l'une quelconque des revendications précédentes, les hauteurs des protubérances étant identiques sur le corps conique du noyau de support.

8. Obturateur dentaire avec support selon les revendications précédentes, dans lequel les protubérances individuelles définissent des points de contact discrets avec la paroi de cavité de canal radiculaire lorsque le corps du noyau de support est inséré dans un canal radiculaire.

9. Obturateur dentaire avec support selon l'une quelconque des revendications précédentes, dans lequel les protubérances individuelles au-dessus de la surface du corps de noyau de support sont discrètes, car elles ne se raccordent pas entre elles pour former une protubérance ressemblant à une bande ou une ligne de contact avec les parois de canal radiculaire.

10. Obturateur dentaire avec support selon l'une quelconque des revendications précédentes, dans lequel les protubérances sont définies comme des protubérances semi-sphériques, semi-ovoïdes, tétraédriques et/ou pyramidales.

11. Obturateur dentaire avec support selon dans l'une quelconque des revendications précédentes, dans lequel le corps de noyau de support présente un seul évasement le long de la section à insérer dans un canal radiculaire.

12. Obturateur dentaire avec support selon l'une quelconque des revendications 1 à 10, dans lequel le corps de noyau de support présente de multiples évasements le long de la direction axiale du corps de noyau de support.

13. Obturateur dentaire avec support selon l'une quelconque des revendications précédentes, dans lequel le corps de noyau de support est constitué d'un matériau qui peut inclure au moins un polymère, tel que le polyéthylène, le polyuréthane, le polychlorure de vinyle.

14. Procédé de fabrication d'un obturateur dentaire avec support (100') comprenant :
la fourniture d'un moule ayant au moins une cavité de moule de forme correspondante à l'obturateur avec support ;
la fourniture d'un noyau de support (10) ayant un corps généralement conique, dans lequel une pluralité de protubérances discrètes (16'a, 16'b, 16'c, 16'd) sont prévues au niveau d'au moins deux emplacements axiaux sur la surface du corps conique ;
et le moulage d'une couche (12) de matériau de remplissage endodontique enrobant le noyau de support, dans lequel l'épaisseur de la couche d'enrobage est environ identique à la hauteur des protubérances au-dessus de la surface du corps conique,
dans lequel les protubérances comprennent une protubérance discrète individuelle au niveau de chaque emplacement axial le long du corps de noyau de support, et dans lequel les positions circonférentielles relatives des protubérances sont décalées d'un angle prédéterminé entre des protubérances axialement adjacentes.
